# EUROPÄISCHE PATENTSCHRIFT

(11) **EP 2 354 133 B1**
(45) Veröffentlichungstag und Bekanntmachung des Hinweises auf die Patenterteilung: **11.12.2013**
(21) Anmeldenummer: 11474001.2
(22) Anmeldetag: 28.01.2011
(51) Int. Cl.: C07D 401/04

(54) **Herstellungsverfahren von 1-(3-Cyano-3,3-diphenylpropyl)-4-(1-piperidyl)-piperidin-4-carboxamid (Piritramid)**
Method for producing 1-(3-Cyano-3,3-diphenylpropyl)-4-(1-piperidyl)-piperidine-4-carboxamide (Piritramide)
Procédé de fabrication de 1-(3-cyano-3,3-diphénylpropyl)-4-(1-pipéridyl)-pipéridine-4-carboxamide (piritramide)

(30) Priorität: 28.01.2010 SK 50022010
(43) Veröffentlichungstag der Anmeldung: 10.08.2011
(73) Patentinhaber: hameln rds gmbh, 31789 Hameln (DE)
(72) Erfinder: Kakalík, Ivan, 90081 SEnkvice (SK); Valachovic, Pavol, 902 01 Pezinok (SK); Smahovský, Vendel, 902 01 Pezinok (SK)

(56) Entgegenhaltungen:
- US-A- 3 080 366
- J. MED. CHEM., Bd. 7, September 1964 (1964-09), Seiten 619-623, XP002631028,

## Beschreibung

### TECHNISCHES GEBIET

Die Erfindung bezieht sich auf ein neues Herstellungsverfahren von 1-(3-Cyano-3,3-diphenylpropyl)-4-(1-piperidyl)-piperidin-4-carboxamid, bekannt unter dem INN-Namen Piritramid. Piritramid ist ein synthetisches opioides Analgetikum.

### STAND DER TECHNIK

Die Herstellung von 1-(3-Cyano-3,3-diphenylpropyl)-4-(1-piperidyl)-piperidin-4-carboxamid (Piritramid) nach Formel III 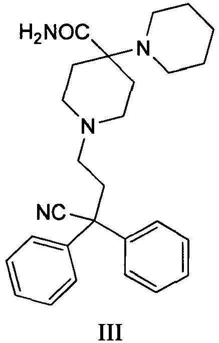 ist in den Patenten US 915835 (1960) und US 3080366 (1963), sowie auch im J. Med. Chem.: 7, 619, (1964) beschrieben. Piritramid wird mittels einer Reaktion von 2 Äquivalenten 4-Brom-2,2-diphenylbutyronitril mit 1 Äquivalent 4-(1-Piperidinyl)-piperidin-4-carboxamid in Toluen unter Verwendung von 1,5 Äquivalenten Natriumcarbonat als Base sowie einer katalytischen Menge von Kaliumjodid (10%) hergestellt. Bei diesem Verfahren wird ein 100%iger Überschuss des Reaktanden 4-Brom-2,2-diphenyl-butyronitril mit dem zweiten Reaktanden 4-(1-Piperidinyl)-piperidin-4-carboxamid eingesetzt.

Ein Nachteil dieser Methode ist einerseits die Verschwendung des Reaktanden, welcher in den meisten Fällen nicht wieder isoliert werden kann, und außerdem die Notwendigkeit der Verwendung eines Katalysators.
Aufgabe dieser Erfindung war es daher ein Verfahren bereitzustellen, mit dem Piritramid ökonomischer, d.h. mit höherer Ausbeute, weniger Nebenprodukten und mit hoher Reinheit synthetisiert werden kann.

Überraschenderweise wurde jetzt festgestellt, dass sich eine größere Ausbeute an Piritramid durch Verwendung eines nur geringen Überschusses (max. 30%) von 4-Brom-2,2-diphenyl-butyronitril und ohne Einsatz eines Katalysators erreichen lässt, wenn ein anderes, als in der Literatur bekanntes, Lösungsmittel verwendet wird. Zusätzlich wurde eine äußerst effektive Isolierung des hochreinen Piritramids aus dem Reaktionsgemisch, über sein Säureadditionssalz und nachfolgender Rücküberführung des Additionssalzes in die Piritramid-Base, entdeckt.

### BESCHREIBUNG DER ERFINDUNG

Die Erfindung betrifft die Herstellung von 1-(3-Cyano-3,3-diphenylpropyl)-4-(1-piperidyl)-piperidin-4-carboxamid (Piritramid) nach Formel III.

1-(3-Cyano-3,3-diphenylpropyl)-4-(1-piperidyl)piperidin-4-carboxamid (Piritramid) wird mittels einer Reaktion von 0,9 bis 1,3 Äquivalenten von 4-Brom-2,2-diphenyl-butyronitril nach Formel I mit 1 Äquivalent 4-(1-Piperidinyl)-piperidin-4-carboxamid nach Formel II in basischer Umgebung und in einem Lösungsmittel wie N,N-Dimethylformamid (DMF) oder einem aliphatischen Alkohol mit einem Siedepunkt im Bereich zwischen 100 und 140°C hergestellt.
In einer bevorzugten Variante der Erfindung liegt das Verhältnis von I zu II zwischen 1 und 1.2 Moläquivalenten. Die besten Ergebnisse lassen sich erzielen, wenn die Verbindung I etwa im Verhältnis 1.1 eingesetzt wird.
Die Isolierung des reinen Piritramids aus dem Reaktionsgemisch kann durch chromatographische Reinigung oder Kristallisation erfolgen, wird aber vorzugsweise über dessen Additionssalz (Formel IV) mit einer anorganischen oder organischen Säure der allgemeinen Formel HX durchgeführt. Das Additionssalz von Piritramid nach Formel IV wird in Form eines kristallinen Stoffs isoliert oder in Wasser extrahiert, wenn das Additionssalz von Piritramid nach Formel IV wasserlöslich ist.
Das Additionssalz des 1-(3-Cyano-3,3-diphenylpropyl)-4-(1-piperidyl)-piperidin-4-carboxamids Formel IV wird anschließend durch Einwirkung einer anorganischen Base im Wasser wieder in 1-(3-Cyano-3,3-diphenylpropyl)-4-(1-piperidyl)-piperidin-4-carboxamid der Formel III überführt.

Piritramid der Formel III wird aus dem Wasser in ein organisches Lösungsmittel extrahiert, wobei die Substanz vorzugsweise nach Verdampfen des organischen Lösungsmittels auskristallisiert wird.
Dieser Aufreinigungsprozess kann auch mit Verbindungen durchgeführt werden, die nicht nach dem in dieser Anmeldung beschriebenen Verfahren hergestellt wurden.

Das gesamte Herstellungsverfahren ist in Schema 1 veranschaulicht.

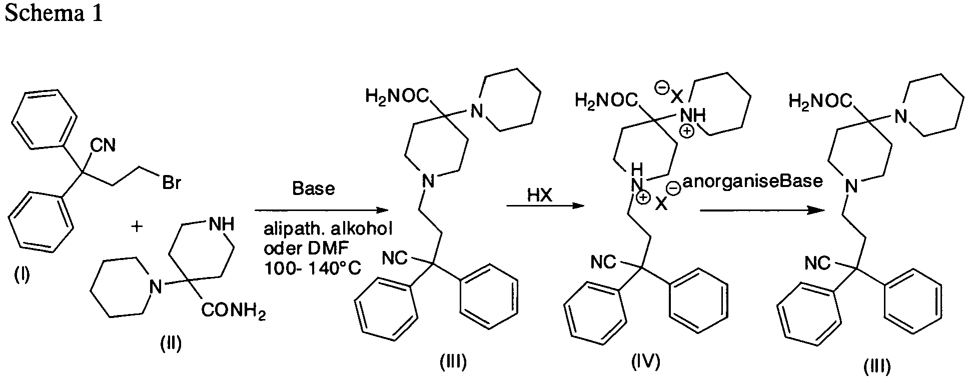

Die Synthese des Piritramids erfolgt beim Siedepunkt des entsprechenden aliphatischen Alkohols oder des *N,N*-Dimethylformamids (DMF) in einem Temperaturbereich zwischen 100 und 140°C. Das Produkt nach Formel III entsteht in einer Ausbeute im Bereich von 82 und 89% (Tabelle 1.), wobei der Reaktionsumsatz gegenüber dem in den Patenten US 915835 (1960) und US 3080366 (1963) beschriebenen Verfahren, bei dem als Lösungsmittel Toluen verwendet wurde, um 10% gesteigert wurde (Tabelle 1). Zusätzlich konnte die Menge des bei der Reaktion eingesetzten Ausgangsstoffs 4-Brom-2,2-diphenyl-butyronitril nach Formel I fast um die Hälfte (siehe Tabelle 1.) reduziert werden. Ebenso war bei der Reaktion die Anwesenheit des Kaliumjodid-Katalysators nicht notwendig.

Bei der Reaktion werden als schwache Basen Kaliumcarbonat, Natriumcarbonat, Natriumhydrogencarbonat, Kaliumhydrogencarbonat, Triethylamin, Pyridin oder Diethylmethylamin eingesetzt.

Die Reaktionszeit, welche zwischen 10 und 30 Stunden liegt, ist von der Reaktionstemperatur abhängig; für die günstigste Temperatur der verwendeten Lösungsmittel beträgt die optimale Reaktionszeit 24 Stunden. Bei der Reaktion werden aliphatische unverzweigte und verzweigte Alkohole als Lösungsmittel mit Siedepunkten zwischen 100 und 140°C verwendet, z.B. 1-Butanol, 2-Butanol, 2-Methyl-1-propanol, 1-Pentanol, 2-Pentanol, 3-Pentanol, 2-Methyl-1-butanol, 3-Methyl-1-butanol, 2-Methyl-2-butanol, 3-Methyl-2-butanol, 2,2-Dimethyl-l-propanol, 4-Methyl-2-pentanol, 2-Ethyl-1-butanol. Zusätzlich kann DMF als Lösungsmittel verwendet werden. Die besten Resultate werden bei Durchführung der Reaktion in 2-Methyl-1-propanol, DMF oder 2-Pentanol erzielt.

Danach wird das Piritramid aus dem Reaktionsgemisch über sein Additionssalz nach Formel IV isoliert, welches durch Beigabe einer anorganischen oder organischen Säure mit der allgemeinen Formel HX in der Rohreaktionsmischung entsteht. Als HX-Säure wird Chlorwasserstoff-, Bromwasserstoff-, Methansulfon-, 4-Toluensulfon-, Benzolsulfon-, Kampfersulfon-, Trifluormethansulfon-, Essig-, Ameisen-, Propion-, Wein-, Bernstein-, Malein-, Oxal-, Glutar- oder Malonsäure eingesetzt.

Das Additionssalz von Piritramid nach Formel IV wird aus dem Rohreaktionsgemisch in Form eines kristallinen Stoffs isoliert. Ist das Additionssalz nach Formel IV wasserlöslich, so wird es ohne vorherige Isolierung in Wasser extrahiert. Das Additionssalz von Piritramid nach Formel IV wird in der wässrigen Lösung einer anorganischen Base wieder in die Base des Piritramids Formel III rücküberführt.

Als anorganische Basen kommen Natriumhydroxid, Kaliumhydroxid, Kaliumcarbonat, Natriumcarbonat, Natriumhydrogencarbonat oder Kaliumhydrogencarbonat zum Einsatz.

Das Piritramid Formel III wird aus dem Wasser in ein organisches Lösungsmittel extrahiert und kristallisiert nach Verdampfen des Lösungsmittels.

Zur Kristallisation lassen sich Methanol, Ethanol, 2-Propanol, Ethylacetat, n-Butylacetat, Aceton und Ethylmethylketon verwenden.

Durch das der vorliegenden Erfindung entsprechende Verfahren wird das Produkt 1-(3-Cyano-3,3-diphenylpropyl)-4-(1-piperidyl)-piperidin-4-carboxamid nach Formel III im Vergleich zu der in gemäß den Patenten US 915835 (1960) und US 3080366 (1963) sowie in J. Med. Chem.: 7, 619, (1964) beschriebenen Methode mit erhöhter Ausbeute und in einem höheren Reinheitsgrad gewonnen. Die Ergebnisse sind in Tabelle 1 angeführt.

Das entsprechend der vorliegenden Erfindung zubereitete Piritramid eignet sich zum Einsatz als pharmazeutische Substanz zur Herstellung eines Arzneimittels in der pharmazeutischen Industrie.

**Tabelle 1.**

| Ausgangsstoffe (Mol-Äquivalente) | | Lösungsmittel Temperatur (°C) Verwendete Base | Isolierung (Reinigung) des Produktes | Reaktionsumsatz (%) | Ausbeute (%) | HPLC-Reinheit (Fläche in %) |
|---|---|---|---|---|---|---|
| I | II | | | | | |
| | | *Toluen* | *A: -* | | | |
| *2 Äquiv.* | *1 Äquiv.* | *111°C* | *B: Aceton^{**}* | *72,9* | *47,5* | *98,4^{*}* |
| | | *Na₂CO₃, KI* | | | | |
| | | 1-Butanol | A: MeCO₂H | | | |
| 1,26 Äquiv. | 1 Äquiv. | 110°C | B: EtCOMe | 86,8 | 65,8 | 99,91 |
| | | NaHCO₃ | | | | |
| | | 2-Methyl-1-propanol, 107°C | A: Weinsäure. | | | |
| 1,05 Äquiv. | 1 Äquiv. | | B: EtCOMe | 82,6 | 66,2 | 99,85 |
| | | K₂CO₃ | | | | |
| | | 3-Pentanol | A: MeSO₃H | | | |
| 1 Äquiv. | 1,1 Äquiv. | 115°C | B: i-PrOH | 88,6 | 74,7 | 99,84 |
| | | NaHCO₃ | | | | |
| | | 2-Methyl-1-propanol, 107°C | A: HCl | | | |
| 1 Äquiv. | 1 Äquiv. | | B: EtOAc | 84,8 | 64,7 | 99,94 |
| | | K₃CO₃ | | | | |
| | | DMF | A: HBr | | | |
| 1,08 Äquiv. | 1 Äquiv. | 120°C | B: n-BuOAc | 83,2 | 62,9 | 99,79 |
| | | Na₂CO₃ | | | | |
| | | 2-Butanol | A: p-PhSO₃H | | | |
| 1,1 Äquiv. | 1 Äquiv. | 99,5°C | B: EtOH | 85,6 | 68 | 99,83 |
| | | KHCO₃ | | | | |
| | | 2-Pentanol | A: MeSO₃H | | | |
| 1,1 Äquiv. | 1 Äquiv. | 119°C | B: Aceton | 87,9 | 72,3 | 99,86 |
| | | Et₃N | | | | |

| | | | | | | |
|---|---|---|---|---|---|---|
| * - Piritramid hergestellt nach dem Verfahren gemäß US-Patent 915835; ** - Das Produkt wurde zweimal aus Aceton kristallisiert. I - 4-Brom-2,2-diphenyl-butyronitril; II - 4-(1-Piperidinyl)-piperidin-4-carboxamid; A - Reinigung mit Salz einer Säure; B - Kristallisation aus einem Lösungsmittel. | | | | | | |

### AUSFÜHRUNGSBEISPIELE

### Beispiel 1

In 50 ml 1-Butanol werden bei einer Temperatur von 110°C über einen Zeitraum von 24 Stunden unter Stickstoff-Atmosphäre 5 g (23,7) 4-(1-Piperidinyl)-piperidin-4-carboxamid, 7,1 g (23,7 mmol) 4-Brom-2,2-diphenyl-butyronitril, 2,5 g (29,8 mmol) Natriumhydrogen-carbonat und 1,6 g (11,2 mmol) Natriumsulfat gemischt. Das Reaktionsgemisch wird auf 25°C abgekühlt und in einem Rotationsverdampfer zur Trockenmasse eingeengt. Das Rohkonzentrat wird in 100 ml destilliertem Wasser und 100 ml Chloroform verrührt, die organische Schicht abgetrennt und mit Natriumsulfat getrocknet. In die Chloroform-Phase werden 2,85 g (47,4 mmol) Essigsäure getropft und das Gemisch wird bei 10°C eine Stunde lang gerührt; das ausgefallene Salz wird abgesaugt und mit 20 ml Chloroform gewaschen. Danach wird das Salz in 100 ml einer 1 N-Natriumhydroxidlösung eingerührt und die freie Base in 120 ml Chloroform extrahiert. Die Chloroformschicht wird mit Natriumsulfat getrocknet und zur Trockenmasse eingeengt. Die ölige Base wird aus 45 ml Ethylmethylketon umkristallisiert. So gewinnt man 6,7 g (65,8%) 1-(3-Cyano-3,3-diphenylpropyl)-4-(1-piperidyl)-piperidin-4-carboxamid mit einem Reinheitsgrad von 99,91% (HPLC).
¹H NMR (300MHz, CDCl₃) δ (ppm): 1.35-1.34 (2H, m), 1.42-1.44 (4H, m), 1.5-1.61 (2H, m), 1.82-1.98 (2H, m), 2.01-2.06 (2H, m), 2.19 (2H, t), 2.36-2.39 (4H, m), 2.52-2.59 (2H, m), 2.65 (2H, t), 6.89 (2H, d, NH₂), 7.30-7.33 (4H, m), 7.38-7.41 (4H, m), 7.42-7.44 (4H, m)
¹³C NMR, δ: 24.68 (CH₂), 26.60 (2xCH₂), 30.35 (2xCH₂), 35.39 (CH₂), 46.84 (2xCH₂N), 49.80 (C), 50.28 (2xCH₂N), 54.40 (CH₂N), 62.71 (C), 122.14 (CN), 126.45 (4xCHₐᵣ), 127.78 (2xCHₐᵣ), 128.90 (4xCHₐᵣ), 140.06 (2xCₐᵣ), 172.63 (CONH₂)
MS: m/e =431 (M+1)⁺

### Beispiel 2

In 45 ml 2-Methyl-1-propanol werden unter Rückfluss in Stickstoff-Atmosphäre über einen Zeitraum von 24 Stunden 5 g (23,7) 4-(1-Piperidinyl)-piperidin-4-carboxamid, 7,1 g (23,7 mmol) 4-Brom-2,2-diphenyl-butyronitril und 4.2 g (30,7 mmol) Kaliumcarbonat gerührt. Das Reaktionsgemisch wird auf 25°C abgekühlt und in einem Rotationsverdampfer zur Trockenmasse eingeengt. Das Rohkonzentrat wird in 100 ml destilliertem Wasser und 100 ml Chloroform verrührt, die organische Schicht wird abgetrennt und mit Natriumsulfat getrocknet. Dann wird sie auf 10°C abgekühlt und die Lösung wird unter Rührung mit 2 g (54.8 mmol) flüssigem Chlorwasserstoff durchgast. Das ausgefallene Dihydrochlorid des 1-(3-Cyano-3,3-diphenylpropyl)-4-(1-piperidyl)piperidin-4-carboxamids wird mit 20 ml Chloroform gewaschen, abgesaugt und in 100 ml 1N Natriumhydroxid in die Base rücküberführt, die in 100 ml Chloroform extrahiert wird. Die organische Schicht wird mit Natriumsulfat getrocknet und eingeengt; das ausgefallene ölige Produkt wird aus 80 ml Ethylacetat auskristallisiert. Man gewinnt 6,6 g (64,7%) 1-(3-Cyano-3,3-diphenylpropyl)-4-(1-piperidyl)piperidin-4-carboxamid mit einem Reinheitsgrad von 99,94% (HPLC).
¹H NMR (300MHz, CDCl₃) δ (ppm): 1.35-1.34 (2H, m), 1.42-1.44 (4H, m), 1.5-1.61 (2H, m), 1.82-1.98 (2H, m), 2.01-2.06 (2H, m), 2.19 (2H, t), 2.36-2.39 (4H, m), 2.52-2.59 (2H, m), 2.65 (2H, t), 6.89 (2H, d, NH₂), 7.30-7.33 (4H, m), 7.38-7.41 (4H, m), 7.42-7.44 (4H, m)
¹³C NMR, δ: 24.68 (CH₂), 26.60 (2xCH₂), 30.35 (2xCH₂), 35.39 (CH₂), 46.84 (2xCH₂N), 49.80 (C), 50.28 (2xCH₂N), 54.40 (CH₂N), 62.71 (C), 122.14 (CN), 126.45 (4xCHₐᵣ), 127.78 (2xCHₐᵣ), 128.90 (4xCHₐᵣ), 140.06 (2xCₐᵣ), 172.63 (CONH₂)
MS: m/e =431 (M+1)⁺

### Beispiel 3

In 150 ml N,N-Dimethylformamid werden bei einer Temperatur von 120°C über einen Zeitraum von 10 Stunden unter Stickstoff-Atmosphäre 15 g (71,0 mmol) 4-(1-Piperidinyl)-piperidin-4-carboxamid, 23 g (76,6 mmol) 4-Brom-2,2-diphenyl-butyronitril sowie 9 g (85,1 mmol) Natriumcarbonat gerührt. Das Reaktionsgemisch wird auf 25°C abgekühlt, die anorganischen Salze werden abgesaugt und in die Lösung wird bei einer Temperatur von 10°C gasförmiger Bromwasserstoff 12 g (148,1 mmol) eingeleitet. Das ausgefallene Dihydrobromid des 1-(3-Cyano-3,3-diphenylpropyl)-4-(1-piperidyl)piperidin-4-carboxamids wird mit 20 ml N,N-Dimethylformamid gewaschen, abgesaugt und durch Einrühren in 250 ml IN Natriumhydroxid in die Base rücküberführt. Die ausgefallene Base wird in 300 ml Dichlormethan extrahiert. Die organische Schicht wird mit Natriumsulfat getrocknet und eingeengt; das ausgefallene ölige Produkt kristallisiert aus 130 ml n-Butylacetat aus. Man bekommt 19,2 g (62,9%) 1-(3-Cyano-3,3-diphenylpropyl)-4-(1-piperidyl)piperidin-4-carboxa-mid mit einem Reinheitsgrad von 99,79% (HPLC).
¹H NMR (300MHz, CDCl₃) δ (ppm): 1.35-1.34 (2H, m), 1.42-1.44 (4H, m), 1.5-1.61 (2H, m), 1.82-1.98 (2H, m), 2.01-2.06 (2H, m), 2.19 (2H, t), 2.36-2.39 (4H, m), 2.52-2.59 (2H, m), 2.65 (2H, t), 6.89 (2H, d, NH₂), 7.30-7.33 (4H, m), 7.38-7.41 (4H, m), 7.42-7.44 (4H, m)
¹³C NMR, δ: 24.68 (CH₂), 26.60 (2xCH₂), 30.35 (2xCH₂), 35.39 (CH₂), 46.84 (2xCH₂N.), 49.80 (C), 50.28 (2xCH₂N), 54.40 (CH₂N), 62.71 (C), 122.14 (CN), 126.45 (4xCHₐᵣ), 127.78 (2xCHₐᵣ), 128.90 (4xCHₐᵣ), 140.06 (2xCₐᵣ), 172.63 (CONH₂)
MS: m/e =431 (M+1)⁺

### Beispiel 4

In 60 ml 2-Butanol werden unter Rückfluss unter Stickstoff-Atmosphäre 30 Stunden lang 20 g (94,6) 4-(1-Piperidinyl)-piperidin-4-carboxamid, 31,3 g (104,1 mmol) 4-Brom-2,2-diphenyl-butyronitril, 2,5 g (122,0 mmol) Kaliumhydrogencarbonat sowie 6,7 g (47,3 mmol) Natriumsulfat gerührt. Das Reaktionsgemisch wird auf 25°C abgekühlt und zur Trockenmasse eingeengt. Das Rohprodukt wird mit 400 ml destilliertem Wasser und 100 ml Dichlormethan verrührt, die organische Schicht wird abgetrennt und mit Natriumsulfat getrocknet. In das Dichlormethan werden 36,0 g (189,2 mmol) des Monohydrats einer p-Toluensulfonsäure getropft und das Gemisch wird bei 10°C eine Stunde gerührt. Das ausgefallene Tosylatsalz wird abgesaugt und mit 50 ml Dichlormethan durchgewaschen. Danach wird das Salz in 400 ml 1N-Natriumhydroxid-Lösung eingerührt und die freie Base in 420 ml Dichlormethan extrahiert. Die Dichlormethanschicht wird mit Natriumsulfat getrocknet und zur Trockenmasse eingeengt. Die ölige Base kristallisiert aus 120 ml Ethanol aus. Man gewinnt 27,7 g (68%) 1-(3-Cyano-3,3-diphenylpropyl)-4-(1-piperidyl)-piperidin-4-carboxamid mit einem Reinheitsgrad von 99,83% (HPLC).
¹H NMR (300MHz, CDCl₃) δ (ppm): 1.35-1.34 (2H, m), 1.42-1.44 (4H, m), 1.5-1.61 (2H, m), 1.82-1.98 (2H, m), 2.01-2.06 (2H, m), 2.19 (2H, t), 2.36-2.39 (4H, m), 2.52-2.59 (2H, m), 2.65 (2H, t), 6.89 (2H, d, NH₂), 7.30-7.33 (4H, m), 7.38-7.41 (4H, m), 7.42-7.44 (4H, m)
¹³C NMR, δ: 24.68 (CH₂), 26.60 (2xCH₂), 30.35 (2xCH₂), 35.39 (CH₂), 46.84 (2xCH₂N), 49.80 (C), 50.28 (2xCH₂N), 54.40 (CH₂N), 62.71 (C), 122.14 (CN), 126.45 (4xCHₐᵣ), 127.78 (2xCHₐᵣ), 128.90 (4xCHₐᵣ), 140.06 (2xCₐᵣ), 172.63 (CONH₂)
MS: m/e =431 (M+1)⁺

### Beispiel 5

In 50 ml 2-Pentanol werden unter Rückfluss unter Stickstoff-Atmosphäre über einen Zeitraum von 28 Stunden 5,1 g (24,1 mmol) 4-(1-Piperidinyl)-piperidin-4-carboxamid, 8 g (26,6 mmol) 4-Brom-2,2-diphenyl-butyronitril sowie 10 ml (71,7 mmol) Triethylamin gerührt. Das Reaktionsgemisch wird auf 25°C abgekühlt und im Rotationsverdampfer zur Trockenmasse eingeengt. Das Rohkonzentrat wird mit 110 ml destilliertem Wasser sowie 100 ml Chloroform vermischt. Die organische Schicht wird abgetrennt und mit Natriumsulfat getrocknet. In das Chloroform werden 4,6 g (48,2 mmol) Methansulfonsäure getropft, das Gemisch wird bei 10°C eine Stunde lang gerührt. Das ausgefallene Mesylatsalz wird abgesaugt und mit 25 ml Chloroform gewaschen. Danach wird das Salz in 100 ml 1N-Natriumhydroxid-Lösung eingerührt und die freie Base wird in 120 ml Chloroform extrahiert. Die Chloroformschicht wird mit Natriumsulfat getrocknet und zur Trockenmasse eingeengt. Das ölige Konzentrat kristallisiert aus 120 ml Aceton aus. Man gewinnt 7,5 g (72,3%) 1-(3-Cyano-3,3-diphenylpropyl)-4-(1-piperidyl)-piperidin-4-carboxamid mit einem Reinheitsgrad von 99,86% (HPLC).
¹H NMR (300MHz, CDCl₃) δ (ppm): 1.35-1.34 (2H, m), 1.42-1.44 (4H, m), 1.5-1.61 (2H, m), 1.82-1.98 (2H, m), 2.01-2.06 (2H, m), 2.19 (2H, t), 2.36-2.39 (4H, m), 2.52-2.59 (2H, m), 2.65 (2H, t), 6.89 (2H, d, NH₂), 7.30-7.33 (4H, m), 7.38-7.41 (4H, m), 7.42-7.44 (4H, m)
¹³C NMR, δ: 24.68 (CH₂), 26.60 (2xCH₂), 30.35 (2xCH₂), 35.39 (CH₂), 46.84 (2xCH₂N), 49.80 (C), 50.28 (2xCH₂N), 54.40 (CH₂N), 62.71 (C), 122.14 (CN), 126.45 (4xCHₐᵣ), 127.78 (2xCHₐᵣ), 128.90 (4xCHₐᵣ), 140.06 (2xCₐᵣ), 172.63 (CONH₂)
MS: m/e =431 (M+1)⁺

### Beispiel 6

In 60 ml 3-Pentanol werden unter Rückfluss unter Stickstoff-Atmosphäre über einen Zeitraum von 24 Stunden 5,4 g (25,6 mmol) 4-(1-Piperidinyl)-piperidin-4-carboxamid, 7 g (23,3 mmol) 4-Brom-2,2-diphenyl-butyronitril, 2,5 g (29,8 mmol) Natriumhydrogencarbonat sowie 1,6 g (11,3 mmol) Natriumsulfat gerührt. Das Reaktionsgemisch wird auf 25°C abgekühlt und in einem Rotationsverdampfer zur Trockenmasse eingeengt. Das Rohkonzentrat wird mit 100 ml destilliertem Wasser und 80 ml Chloroform gemischt, die organische Schicht wird abgetrennt und mit 30 ml Wasser durchgewaschen. Dem Chloroform werden unter intensivem Rühren 4,9 g (51 mmol) Methansulfonsäure aufgelöst in 90 ml destilliertem Wasser zugefügt; danach wird das Gemisch bei 30° C 20 Minuten lang intensiv gerührt. Die wässrige Schicht wird abgetrennt und mit 20 ml Chloroform durchgewaschen. Danach werden 26 ml einer 1N-Natriumhydroxid-Lösung zugegeben. Die freie Base wird in 3 x 50 ml Chloroform extrahiert, die vereinigten organischen Schichten werden mit Natriumsulfat getrocknet und zur Trockenmasse eingeengt. Das ölige Produkt kristallisiert aus 40 ml 2-Propanol. Man erhält 7,5 g (74,7%) 1-(3-Cyano-3,3-diphenylpropyl)-4-(1-piperidyl)-piperidin-4-carboxamid mit einem Reinheitsgrad von 99,84% (HPLC).
¹H NMR (300MHz, CDCl₃) δ (ppm): 1.35-1.34 (2H, m), 1.42-1.44 (4H, m), 1.5-1.61 (2H, m), 1.82-1.98 (2H, m), 2.01-2.06 (2H, m), 2.19 (2H, t), 2.36-2.39 (4H, m), 2.52-2.59 (2H, m), 2.65 (2H, t), 6.89 (2H, d, NH₂), 7.30-7.33 (4H, m), 7.38-7.41 (4H, m), 7.42-7.44 (4H, m)
¹³C NMR, δ: 24.68 (CH₂), 26.60 (2xCH₂), 30.35 (2xCH₂), 35.39 (CH₂), 46.84 (2xCH₂N), 49.80 (C), 50.28 (2xCH₂N), 54.40 (CH₂N), 62.71 (C), 122.14 (CN), 126.45 (4xCHₐᵣ), 127.78 (2xCHₐᵣ), 128.90 (4xCHₐᵣ), 140.06 (2xCₐᵣ), 172.63 (CONH₂)
MS: m/e =431 (M+1)⁺

### Beispiel 7

In 60 ml 2-Methyl-l-Propanol werden unter Rückfluss unter Stickstoff-Atmosphäre 24 Stunden lang 6,3 g (29,8 mmol) 4-(1-Piperidinyl)-piperidin-4-carboxamid, 9,4 g (31,3 mmol) 4-Brom-2,2-diphenyl-butyronitril und 5,3 g (38,7 mmol) Kaliumcarbonat gerührt. Das Reaktionsgemisch wird auf 25°C abgekühlt und zur Trockenmasse eingeengt. Der Rohrückstand wird mit 100 ml destilliertem Wasser sowie 110 ml 1,2 Dichlorethan vermischt, die organische Schicht wird abgetrennt und mit 40 ml Wasser gewaschen. Der 1,2 Dichlorethan-Lösung werden unter intensivem Rühren 8,9 g (59,6 mmol) Weinsäure, aufgelöst in 250 ml destilliertem Wasser, zugefügt; danach wird das Gemisch bei 30°C 20 Minuten lang intensiv gerührt. Die wässrige Schicht wird abgetrennt, mit 40 ml 1,2 Dichlorethan gewaschen und danach 30 ml einer 1N-Natriumhydroxid-Lösung zugesetzt. Die freie Base wird in 3 x 60 ml 1,2 Dichlorethan extrahiert, die vereinigten organischen Phasen werden mit Natriumsulfat getrocknet und zur Trockenmasse eingeengt. Das ölige Produkt wird aus 80 ml Ethylmethylketon umkristallisiert. Man erhält 8,5 g (66,2%) 1-(3-Cyano-3,3-diphenylpropyl)-4-(1-piperidyl)-piperidin-4-carboxamid mit einem Reinheitsgrad von 99,85% (HPLC).
¹H NMR (300MHz, CDCl₃) δ (ppm): 1.35-1.34 (2H, m), 1.42-1.44 (4H, m), 1.5-1.61 (2H, m), 1.82-1.98 (2H, m), 2.01-2.06 (2H, m), 2.19 (2H, t), 2.36-2.39 (4H, m), 2.52-2.59 (2H, m), 2.65 (2H, t), 6.89 (2H, d, NH₂), 7.30-7.33 (4H, m), 7.38-7.41 (4H, m), 7.42-7.44 (4H, m)
¹³C NMR, δ: 24.68 (CH₂), 26.60 (2xCH₂), 30.35 (2xCH₂), 35.39 (CH₂), 46.84 (2xCH₂N), 49.80 (C), 50.28 (2xCH₂N), 54.40 (CH₂N), 62.71 (C), 122.14 (CN), 126.45 (4xCHₐᵣ), 127.78 (2xCHₐᵣ), 128.90 (4xCHₐᵣ), 140.06 (2xCₐᵣ), 172.63 (CONH₂)
MS: m/e =431 (M+1)⁺

### INDUSTRIELLE VERWERTBARKEIT

Die Erfindung ist für die Herstellung der pharmazeutischen Substanz Piritramid und der Injektionsform, welche diese Substanz enthält, industriell verwertbar.

## Patentansprüche

1. Verfahren zur Herstellung von 1-(3-Cyano-3,3-Diphenylpropyl)-4-(1-piperidyl)-piperidin-4-carboxamid der Formel III durch Umsetzen von 4-(1-Piperidinyl)-piperidin-4-carboxamid der Formel II mit 4-Brom-2,2-diphenylbutyronitril der Formel I, so dass 1-(3-Cyano-3,3-Diphenylpropyl)-4-(1-piperidyl)-piperidin-4-carboxamid entsteht, wobei die Reaktion in Gegenwart eines aliphatischen Alkohols, oder DMF durchgeführt wird.

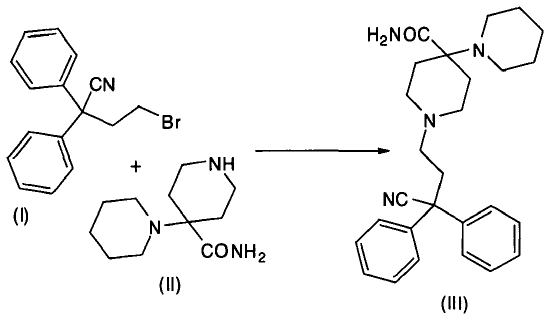

2. Verfahren zur Herstellung der Verbindung III nach Anspruch1, wobei die Reaktion in Gegenwart eines Lösungsmittels durchgeführt wird, ausgewählt aus aliphatischen Alkoholen mit einem Siedepunkt zwischen 100 und 140 °C oder N, N-Dimethylformamid (DMF).

3. Verfahren nach Anspruch 1 oder 2, wobei 1-Butanol, 2-Butanol, 2-Methyl-1-propanol, 1-Pentanol, 2-Pentanol, 3-Pentanol, 2-Methyl-1-butanol, 3-Methyl-1-butanol, 2-Methyl-2-butanol, 3-Methyl-2-butanol, 2,2-Dimethyl-1-propanol, 4-Methyl-2-pentanol oder 2-Ethyl-1-butanol als Lösungsmittel eingesetzt wird.

4. Verfahren zur Herstellung der Verbindung III nach einem der Ansprüche 1 bis 3, wobei die Reaktion in Gegenwart eines Lösungsmittels ausgewählt aus N,N-Dimetylformamid (DMF), 2-Methyl-1-propanol und 2-Pentanol durchgeführt wird.

5. Verfahren zur Herstellung der Verbindung III nach einem der Ansprüche 1 bis 4, wobei die Reaktionspartner in einem Verhältnis (I zu II) zwischen 0,8 und 1,3 eingesetzt werden.

6. Verfahren zur Herstellung der Verbindung III nach einem der Ansprüche 1 bis 5, wobei die Reaktionspartner (I zu II) in einem Verhältnis zwischen 1.0 und 1.2 eingesetzt werden.

7. Verfahren zur Herstellung der Verbindung III nach einem der Ansprüche 1 bis 6, wobei die Verbindung I in einem Überschuss von etwa 10 % eingesetzt wird.

8. Verfahren zur Herstellung der Verbindung III nach einem der vorigen Ansprüche in Abwesenheit eines Katalysators.

9. Verfahren zur Aufreinigung eines Rohprodukts der Formel III umfassend die Schritte:
a. Behandlung der Verbindung III mit einer organischen oder anorganischen Säure der allgemeinen Formel HX zur Bildung eines Additionssalzes von 1 - (3-Cyano-3,3-Diphenylpropyl) -4 - (1-piperidyl)-piperidin-4-carboxamid
b. Isolierung des Säureadditionssalzes durch Kristallisation oder durch Extraktion mit Wasser,
c. Umwandlung des Säureadditionssalzes von 1-(3-Cyano-3,3-Diphenylpropyl)-4- (1-piperidyl)-piperidin-4-carboxamid in die freie Base mit Hilfe einer Base,
d. Extraktion von 1-(3-Cyano-3,3-Diphenylpropyl)-4-(1-piperidyl)-piperidin-4-carboxamid mit einem oder mehreren organischen Lösungsmitteln

10. Verfahren zur Reinigung eines Rohprodukts der Formel III nach Anspruch 9 wobei das Produkt anschließend aus einem organischen Lösungsmittel kristallisiert wird.

11. Verfahren nach Anspruch 9 bei dem als Base eine Verbindung ausgewählt aus der Gruppe Natriumhydroxid, Kaliumhydroxid, Kaliumcarbonat, Natriumcarbonat, Natriumhydrogencarbonat, Kaliumhydrogencarbonat verwendet wird

12. Verfahren nach Anspruch 9, wobei die organische oder anorganische Säure der allgemeinen Formel HX ausgewählt ist aus Salzsäure, Bromwasserstoffsäure, Methansulfonsäure, p-Toluolsulfonsäure, Benzolsulfonsäure, Camphersulfonsäure, Trifluormethansulfonsäure, Essigsäure, Ameisensäure, Propionsäure, Weinsäure, Bernsteinsäure, Oxalsäure, Maleinsäure, Glutarsäure oder Malonsäure.

13. Verfahren nach Anspruch 9 wobei die Säure der allgemeinen Formel HX Methansulfonsäure ist.

14. Verfahren nach Anspruch 10 wobei Methanol, Ethanol, 2-Propanol, Ethylacetat, n-Butylacetat, Aceton oder Ethylmethylketon als Lösungsmittel für die Kristallisation von 1-(3-Cyano-3,3-Diphenylpropyl)-4-(1-piperidyl)-piperidin-4-carboxamid der Formel III verwendet wird.

15. Verfahren zur Herstellung von 1-(3-Cyano-3,3-diphenyl-propyl)-4-(1-piperidyl)piperidin-4-carboxamid Formel III nach einem der oben genannten Ansprüche **dadurch gekennzeichnet, dass** 1 Äquivalent 4-(1-Piperidinyl)-piperidin-4-carboxamid der Formel II mit 0,9 bis 1,3 Äquivalenten 4-Brom-2,2-diphenyl-butyronitril der Formel I bei einer Temperatur von 100 bis 140°C in basischem Milieu sowie unter Verwendung des Lösungsmittels *N,N-*Dimethylformamid oder einem aliphatischen Alkohol, dessen Siedepunkt im Bereich zwischen 100 und 140°C liegt, reagiert, wobei das entstandene Rohprodukt 1-(3-Cyano-3,3-diphenyl-propyl)-4-(1-piperidyl)-piperidin-4-carboxamid Formel III durch eine organische oder anorganische Säure mit der allgemeinen Formel HX in ein Additionssalz von 1-(3-Cyano-3,3-diphenyl-propyl)-4-(1-piperidyl)-piperidin-4-carboxamid mit der Formel IV überführt wird, das in Form eines kristallinen Stoffes isoliert oder in Wasser extrahiert wird; danach wird das Additionssalz des 1-(3-Cyano-3,3-diphenyl-propyl)-4-(1-piperidyl)-piperidin-4-carboxamids mit der Formel IV durch Einwirken einer anorganischen Base in wässrigem Milieu in die Base des 1-(3-Cyano-3,3-diphenyl-propyl)-4-(1-piperidyl)-piperidin-4-carboxamids der Formel III rücküberführt, welche aus dem Wasser in ein organisches Lösungsmittel zurückextrahiert wird. Nach dem Einengen kristallisiert das Produkt 1-(3-Cyano-3,3-diphenyl-propyl)-4-(1-piperidyl)-piperidin-4-carboxamid Formel III aus einem organischen Lösungsmittel aus.

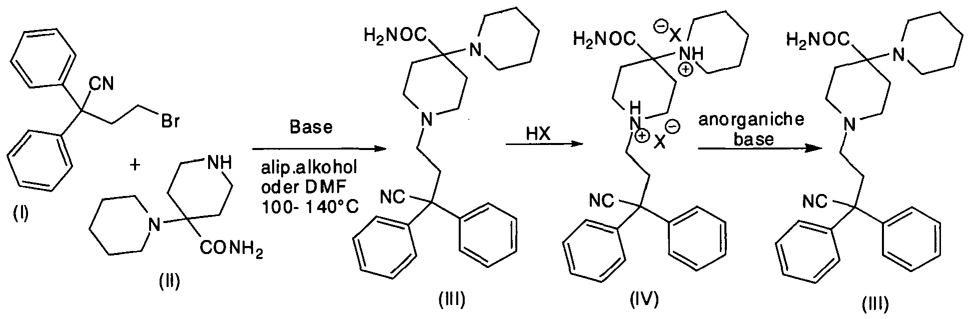

## Claims

1. A method of preparation of 1-(3-cyano-3,3-diphenylpropyl)-4-(1-piperidyl)-piperidin-4-carboxamide of formula III by reacting 4-(1-piperidinyl)-piperidin-4-carboxamide of formula II with 4-brom-2,2-diphenylbutyronitril of formula I forming 1-(3-cyano-3,3-diphenylpropyl)-4-(1-piperidyl)-piperidin-4-carboxamide of formula III wherein the reaction is carried out in the presence of an aliphatic alcohol or N,N-dimethlformamide.

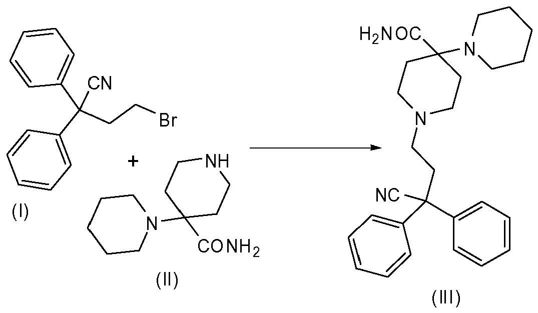

2. A method of preparation of compound III according to claim 1, wherein the reaction is carried out in a solvent selected from aliphatic alcohols with boiling points from 100 to 140°C or N,N-dimetylformamide (DMF).

3. A method of preparation of compound III according to claim 1 or 2, wherein the reaction is carried out in a solvent selected from 1-butanol, 2-butanol, 2-methyl-1-propanol, 1-pentanol, 2-pentanol, 3-pentanol, 2-methyl-1-butanol, 3-methyl-l-butanol, 2-methyl-2-butanol, 3-methyl-2-butanol, 2,2-dimethyl-1-propanol, 4-methyl-2-pentanol or 2-ethyl-1-butanol.

4. A method of preparation of compound III according to one of the preceding claims, wherein the reaction is carried out in a solvent selected from N,N-dimetylformamide (DMF), 2-methyl-1-propanol and 2-pentanol.

5. A method of preparation of compound III according to one of the preceding claims
wherein the ratio of compound I and II is between 0.8 and 1.3.

6. A method of preparation of compound III according to one of the preceding claims wherein the ratio of compound I and II is between 1.0 and 1.2.

7. A method of preparation of compound III according to one of the preceding claims wherein compound I is used in an overage of about 10 %.

8. A method of preparation of compound III according to one of the preceding claims in the absence of a catalyst.

9. A method of purification of a crude compound III comprising the following steps:
a. Treatment of compound III with an organic or inorganic acid of general formula HX to form the additive salt of 1-(3-cyano-3,3-diphenylpropyl)-4-(1-piperidyl)-piperidin-4-carboxamide
b. isolation of the acid addition salt by crystallization or by extraction with water,
c. conversion of the acid addition salt of 1-(3-cyano-3,3-diphenylpropyl)-4-(1-piperidyl)-piperidin-4-carboxamide to the free base in basic aqueous solution
d. extraction of 1-(3-cyano-3,3-diphenylpropyl)-4-(1-piperidyl)-piperidin-4-carboxamide with organic solvent

10. A method of purification of a crude compound III according to claim 9 with subsequent crystallization of the product in an organic solvent.

11. The method according to claim 9 wherein sodium hydroxide, potassium hydroxide, potassium carbonate, sodium carbonate, sodium bicarbonate, potassium bicarbonate, is used as a base.

12. The method according to claim 9, wherein the organic or inorganic acid of general formula HX is hydrochloric acid, hydrobromic acid, methanesulfonic acid, p-toluenesuphonic acid, benzenesulphonic acid, camphorsulphonic acid, trifluoromethanesulfonic acid, acetic acid, formic acid, propionic acid, tartaric acid, succinic acid, oxalic acid, maleic acid, glutaric acid or malonic acid.

13. The method according to claim 9, wherein the organic or inorganic acid of general formula HX is methanesulfonic acid.

14. The method according to claim 10 wherein that methanol, ethanol, 2-propanol, ethylacetat, n-buthylacetat, aceton or ethylmethylketone is used as solvent for crystallization of 1-(3-cyano-3,3-diphenylpropyl)-4-(1-piperidyl)-piperidin-4-carboxamide of formula III.

15. A method of preparation of 1-(3-cyano-3,3-diphenylpropyl)-4-(1-piperidyl)-piperidin-4-carboxamide of formula III according to one of the preceding claims **characterized in that** one equivalent of 4-(1-piperidinyl)-piperidin-4-carboxamide of formula II is reacting with 0.9 to 1.3 equivalents of 4-bromo-2,2-diphenylbutyronitril of formula I at a temperature of 100 to 140 °C in basic solution using an aliphatic alcohol with a boiling point of between 100 and 140 °C or N,N-dimethlformamide as the solvent, wherein the crude product 1-(3-cyano-3,3-diphenylpropyl)-4-(1-piperidyl)-piperidin-4-carboxamide of formula III is forming an addition salt of 1-(3-cyano-3,3-diphenylpropyl)-4-(1-piperidyl)-piperidin-4-carboxamide of formula IV with an organic or inorganic acid of general formula HX which is isolated as a crystalline powder or extracted with water; subsequently, the addition salt of 1-(3-cyano-3,3-diphenylpropyl)-4-(1-piperidyl)-piperidin-4-carboxamide of formula IV is converted in basic solution to the base of 1-(3-cyano-3,3-diphenylpropyl)-4-(1-piperidyl)-piperidin-4-carboxamide of formula III which is extracted from the aqueous solution into an organic solvent. After concentration of the organic solution the product 1-(3-cyano-3,3-diphenylpropyl)-4-(1-piperidyl)-piperidin-4-carboxamide of formula III is crystallizing from the organic solvent.

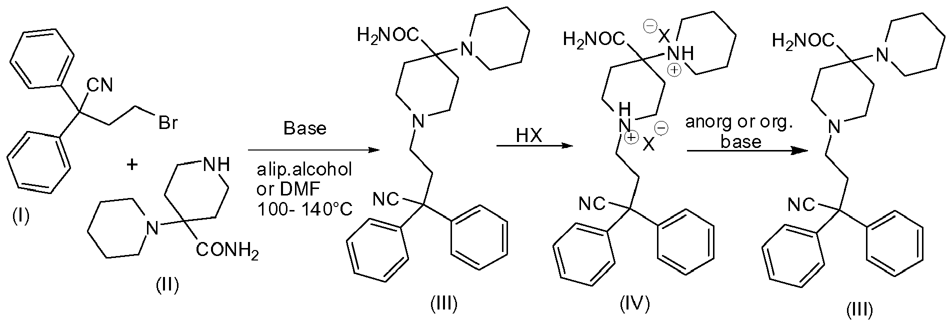

## Revendications

1. Procédé pour la synthèse de 1-(3-cyano-3,3-diphénylpropyl)-4-(1-pipéridyl )pipéridine-4-carboxamide de formule III par la réaction de la 4-(pipéridinyl)-4-carboxamide de formule II avec du 4-bromo-2,2-diphénylbutyronitril de formule I, de sorte que la 1-(3-cyano-3,3-diphénylpropyl)-4-(1-pipéridyl) pipéridine-4-carboxamide est produite. La réaction est effectuée en présence d'un alcool aliphatique ou le DMF.

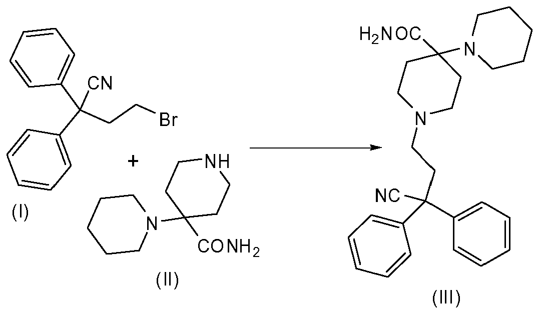

2. Procédé selon la revendication 1 pour la synthèse du composé III, dans lequel la réaction est effectué en présence d'un solvant choisi parmi les alcools aliphatique ayant un point d'ébullition compris entre 100 et 104 °C ou le N,N-diméthylformamide (DMF).

3. Procédé selon la revendication 1 ou 2, dans lequel le 1-butanol, le 2-butanol, le 2-méthyl-1-propanol, le 1-pentanol, le 2-pentanol, le 3-pentanol, le 2-méthyl-1-butanol, le 3-méthyl-1-butanol, le 2-méthyl-2-butanol, le 3-méthyl-2-butanol, le 2,2-diméthyl-1-propanol, le 4-méthyl-2-pentanol ou le 2-éthyl-1-butanol est utilisé comme solvant.

4. Procédé selon l'une quelconque des revendications précédentes, dans lequel la réaction est effectué en présence d'un solvant, qui est choisi dans le groupe constitué du N,N-diméthylformamide (DMF), le 2-méthyl-1-propanole et le 2-pentanole.

5. Procédé selon l'une quelconque des revendications 1 à 4 pour la synthèse du composé III, dans lequel les réactifs sont utilisés dans un rapport (I à II) de 0.8 à 1.3.

6. Procédé selon l'une quelconque des revendications 1 à 5 pour la synthèse de composé III, dans lequel les réactifs sont utilisé dans un rapport de 1.0 à 1.2.

7. Procédé selon l'une quelconque des revendications 1 à 6 pour la synthèse du composé III, dans lequel le composé I est utilisé dans un excès d'environ 10 %.

8. Procédé selon l'une quelconque des revendications précédentes pour la synthèse de composé III en l'absence d'un catalyseur.

9. Procédé pour la purification d'un produit brut de formule III, comprenant les étapes consistant à :
a. Traitement du composé III avec un acide organique ou minéral de la formule générale HX pour former un sel d'addition de 1-(3-cyano-3,3-diphénylpropyl)-4-(1-pipéridyl)pipéridine-4-carboxamide
b. Isolation du sel d'addition d'acide par cristallisation ou par extraction avec de l'eau.
c. Conversion du sel d'addition d'acide de 1-(3-cyano-3,3-diphénylpropyl)-4-(1-pipéridyl)pipéridine-4-carboxamide dans la base libre à l'aide d'une base
d. Extraction du composé III (1-(3-cyano-3,3-diphénylpropyl)-4-(1-pipéridyl)pipéridine-4-carboxamide) avec un ou plusieurs solvants organiques.

10. Procédé pour la purification d'un produit brut de formule III selon la revendication 9, dans lequel le produit est ensuite cristallisé dans un solvant organique.

11. Procédé selon la revendication 9, dans lequel un composé choisi dans le groupe qui suit est utilisé en tant que base : l'hydroxide de sodium, l'hydroxide de potassium, le carbonate de potassium, le carbonate de sodium, le carbonate acide de sodium ou le carbonate acide de potassium.

12. Procédé selon la revendication 9, dans lequel l'acide organique ou minérale de la formule générale HX est choisi dans le groupe formé par l'acide chlorhydrique, l'acide bromhydrique, l'acide méthanesulfonique, l'acide p-toluène-sulfonique, l'acide benzène sulfonique, l'acide camphersulfonyle, l'acide trifluorométhanesulfonique, l'acide acétique, l'acide formique, l'acide propionique, l'acide tartrique, l'acide succinique, l'acide oxalique, l'acide maléique, l'acide glutarique et l'acide malonique.

13. Procédé selon la revendication 9, dans lequel l'acide de formule générale HX est l'acide méthanesulfonique.

14. Procédé selon la revendication 10, dans lequel le solvant pour la cristallisation du composé III (1-(3-cyano-3,3-diphénylpropyl)-4-(1-pipéridyl)pipéridine-4-carboxamide) est choisi dans le groupe consistant en le méthanol, l'éthanol, le propanol-2, l'acétate d'éthyle, l'acétate de n-butyle, l'acétone et la méthyléthylcétone.

15. Procédé pour la synthèse du composé III (1-(3-cyano-3,3-diphénylpropyl)-4-(1-pipéridyl)pipéridine-4-carboxamide) selon l'une quelconque des revendications précédentes, **caractérisé en ce que** 1 équivalent de 4-(1-pipéridinyl)-pipéridine-4-carboxamide de formule II est réagi avec 0.9 à 1.3 équivalents de 4-bromo-2,2-diphényl butyronitrile de formule I à un température de 100 à 140 °C dans un milieu basique et en utilisant comme solvant le N,N-diméthylformamide ou un alcool aliphatique, dont le point d'ébullition est entre 100 à 140 °C, de manière que le produit brut qui est formé (1-(3-cyano-3,3-diphénylpropyl)-4-(1-pipéridyl)pipéridine-4-carboxamide) de formule III est après transformé avec un acide organique ou minéral de la formule générale HX dans un sel d'addition de (1-(3-cyano-3,3-diphénylpropyl)-4-(1-pipéridyl)pipéridine-4-carboxamide) de formule IV, qui est isolé sous forme d'une matière cristalline ou qui est extrait dans de l'eau. Par la suite le sel d'addition de 1-(3-cyano-3,3-diphényl-propyl)-4-(1-pipéridyl)pipéridine-4-carboxamide de formule IV est retransformé dans le base de 1-(3-cyano-3,3-diphényl-propyl)-4-(1-pipéridyl)pipéridine-4-carboxamide de formule III par réaction avec une base minérale en milieu aqueux, qui est re-extrait de l'eau par un solvant organique. Après évaporation, le produit 1-(3-cyano-3,3-diphényl-propyl)-4-(1-pipéridyl)pipéridine-4-carboxamide de formule III est cristallisé d'un solvant organique.
